(19) Europäisches Patentamt
European Patent Office
Office européen des brevets

(11) Veröffentlichungsnummer: **0 137 441**
**A2**

(12) # EUROPÄISCHE PATENTANMELDUNG

(21) Anmeldenummer: **84111745.0**

(22) Anmeldetag: **02.10.84**

(51) Int. Cl.[4]: **C 07 D 501/46**
A 61 K 31/545

(30) Priorität: **08.10.83 DE 3336757**

(43) Veröffentlichungstag der Anmeldung:
**17.04.85 Patentblatt 85/16**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lattrell, Rudolf, Dr.**
**Heuhohlweg 6h**
**D-6240 Königstein/Taunus(DE)**

(72) Erfinder: **Blumbach, Jürgen, Dr.**
**Manderscheider Strasse 13b**
**D-6000 Frankfurt am Main 71(DE)**

(72) Erfinder: **Dürckheimer, Walter, Dr.**
**Im Lerchenfeld 45**
**D-6234 Hattersheim am Main(DE)**

(72) Erfinder: **Schwab, Wilfried, Dr.**
**Am Flachsland 18**
**D-6233 Kelkheim (Taunus)(DE)**

(72) Erfinder: **Seibert, Gerhard, Dr.**
**Gläserweg 21**
**D-6100 Darmstadt(DE)**

(54) **Cephalosporinderivate und Verfahren zu ihrer Herstellung.**

(57) Cephalosporinderivate der allgemeinen Formel

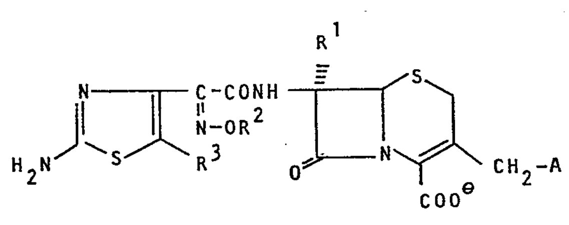

gegen bakterielle Infektionen wirksame pharmazeutische Präparate, die solche Cephemderivate enthalten, Verfahren zur Herstellung der Cephemderivate und der pharmazeutischen Präparate, Verwendung der Cephemderivate zur Bekämpfung bakterieller Infektionen.

EP 0 137 441 A2

# Cephalosporinderivate und Verfahren zu ihrer Herstellung

Die Erfindung betrifft neue Cephalosporinderivate und Verfahren zu ihrer Herstellung, insbesondere polare Cephemderivate, die in 3'-Stellung des Cephemrings durch eine quaternäre Ammoniogruppe substituiert sind und die eine sehr gute antimikrobielle Wirkung gegen gram-positive und gram-negative Bakterien besitzen und deshalb als Arzneimittel zur Behandlung von mikrobiellen Infektionen geeignet sind.

Gegenstand der Erfindung sind daher Cephemderivate der allgemeinen Formel I

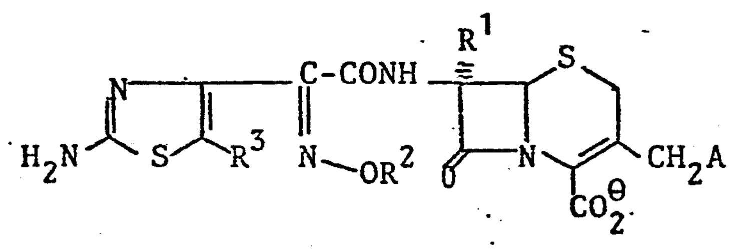

(I)

und deren physiologisch verträgliche Säureadditionssalze worin

$R^1$ Wasserstoff oder Methoxy

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $(CH_2)_n-\underset{R^5}{\overset{R^4}{(C)_m}}-R^6$, worin m und n jeweils für O oder 1 steht und

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfach substituiert sein können,

$R^6$ eine COOH-; CN- oder $CONH_2$-Gruppe,

$R^3$ Wasserstoff oder Halogen und

A ein ungesättigtes, gegebenenfalls substituiertes 5-6-gliedriges aromatisches heterocyclisches Kation der Formel $-\overset{\oplus}{N}$, das weitere N, O oder S-Atome im Ring enthalten kann und an das zusätzliche Ringe ankondensiert sein können, wobei jedoch Pyridinio und seine kondensierten Systeme ausgeschlossen sind, bedeutet, und in der die $R^2$ O-Gruppe in syn-Position steht.

Die vorliegende Erfindung ist insbesondere auf Verbindungen gerichtet, in denen $R^1$ und $R^3$ die vorstehenden Bedeutungen besitzen und

$R^2$ Wasserstoff, $C_1$-$C_6$-Alkyl, das ein- oder mehrfach substituiert sein kann durch Halogen, $C_1$-$C_6$-Alkylthio, $C_1$-$C_6$-Alkoxy, Aryl oder Heteroaryl, $C_2$-$C_6$-Alkenyl, das ein- oder mehrfach substituiert sein kann durch Halogen; $C_2$-$C_3$-Alkinyl, $C_3$-$C_6$-Cycloalkyl, $C_3$-$C_6$-Cycloalkyl-$C_1$-$C_6$-alkyl, $C_4$-$C_7$-Cycloalkenyl und in der die Gruppe

$(CH_2)_n\underset{R^5}{\overset{R^4}{(C)_m}}R^6$ die vorstehende Bedeutung hat;

0137441

A ein heterocyclisches Kation der Formel 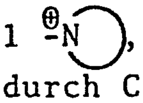, wie z.B. ein 1,2,3- oder 1,2,4-Triazolio, die durch $C_1$-$C_4$-Alkyl substituiert sein können; ein gegebenenfalls mit $C_1$-$C_4$-Alkyl substituiertes Imidazolio, Pyrrolio, Indolio oder Pyrazolio; ein Pyrimidinio, das gegebenenfalls mit Amino oder Halogen substituiert ist; ein Pyridazinio oder Pyrazinio, die durch Halogen oder durch $C_1$-$C_4$-Alkyl substituiert sein können und wobei zwei orthoständige Alkylgruppen auch zu einem gegebenenfalls substituierten Di-bis Heptamethylenring geschlossen sein können; ein Thiazolio, Isothiazolio oder Oxazolio, die durch Hydroxy-$C_1$-$C_4$-Alkyl und/oder $C_1$-$C_4$-Alkyl substituiert sein können; ein Phthalazinio, Chinazolinio, Chinoxalinio, Cinnolinio; und wobei auch bei diesen bevorzugten, unter die allgemeine Formel I fallenden Verbindungen die $R^2$O-Gruppe in syn-Position steht.

Als besonders bevorzugt kommen beispielsweise die folgenden Substituenten in Betracht:

$R^1$: Wasserstoff, Methoxy;

$R^3$: Wasserstoff, Fluor, Chlor, Brom,

$R^2$: Wasserstoff, $C_1$-$C_6$-Alkyl, wie z.B. Methyl, Ethyl, Propyl, Isopropyl, Butyl, vorzugsweise Methyl, Ethyl; $C_1$-$C_2$-Halogenalkyl, z.B. Chlor, Brom, Jod oder Fluor-substituiertes Alkyl, vorzugsweise Trifluorethyl, Difluormethyl, 2,2,3,3,-Tetrafluorpropyl; durch $C_1$-$C_2$-Alkyloxy-$C_1$-$C_2$-alkyl, z.B. Äthoxyäthyl; $C_1$-$C_2$-Alkylmercapto-$C_1$-$C_2$-alkyl, z.B. Methylmercaptomethyl; durch Aryl, wie z.B. Phenyl, Tolyl, Chlorphenyl, substituiertes Alkyl, insbesonders Benzyl;
durch Heteroaryl substituiertes Alkyl, wie z.B. 1,3-Thiazol-4-yl-substituiertes Alkyl, insbesondere 1,3-Thiazol-4-yl-methyl,

$C_2$-$C_6$-Alkenyl, wie z.B. Vinyl, Allyl, Isopropenyl, Methallyl, insbesondere Allyl, Methallyl; durch Halogen, wie z.B. durch Chlor oder Brom substituiertes $C_2$-$C_6$-Alkenyl, insbesondere 3-Chlorpropen-2-yl, 2-Brompropen-2-yl, 2-Chlorpropen-2-yl; $C_2$-$C_3$-Alkinyl, wie insbesondere Propargyl;$C_3$-$C_7$-Cycloalkyl, wie insbesondere Cyclopropyl, Cyclobutyl, Cyclopentyl; $C_3$-$C_7$-Cycloalkyl-$C_1$-$C_6$-alkyl, wie insbesondere Cyclopropylmethyl, Cyclobutylmethyl;
$C_4$-$C_7$-Cycloalkenyl, wie insbesondere Cyclopenten-1-yl;
die Gruppe $(CH_2)_n-(\overset{R^4}{\underset{R^5}{C}})_m-R^6$, wobei $R^6$ die Gruppe COOH, CN oder $CONH_2$ bedeutet und $R^4$ und $R^5$ gleich oder verschieden sein können und Wasserstoff, Aryl, vorzugsweise Phenyl; $C_1$-$C_4$-Alkyl, wie z.B. Methyl, Ethyl, Propyl, Isopropyl, Butyl, sec. Butyl, vorzugsweise Methyl, Ethyl, insbesondere Methyl, bedeuten können oder wobei $R^4$ und $R^5$ zusammen mit dem Kohlenstoffatom, an das sie gebunden sind, eine Methylengruppe oder eine $C_3$-$C_7$-Cycloalkylidengruppe bilden könnnen, wie z.B. Cyclopropyl, Cyclobutyl, Cyclopentyl, Cyclohexyl, Cycloheptyl, vorzugsweise Cyclopropyl, Cyclobutyl, Cyclopentyl oder Cyclohexyl, und wobei die Cycloalkylidengruppe substituiert sein kann, z.B. durch $C_1$-$C_4$-Alkyl, vorzugsweise Methyl, oder durch Halogen, vorzugsweise Fluor und Chlor,

m = 0 oder 1

n = 0 oder 1, wobei die Summe von m und n 1 oder 2 darstellt.

Bevorzugte Beispiele für die Gruppe- $(CH_2)_n(\overset{R^4}{\underset{R^5}{C}})_m$- sind die folgenden:

Für den Fall, daß n = 0 und m = 1 ist; -ĊH($CH_3$), -Ċ($CH_3$)$_2$, ĊH($C_6H_5$),

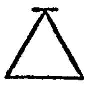 , 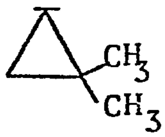 , 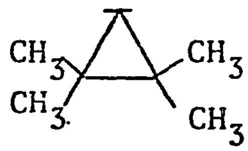 , 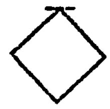 ,

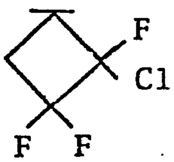 , 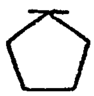 , 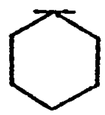 ,

für den Fall, daß m = 0 und n = 1 ist: $-CH_2-$ und falls n und m = 1 sind: $-CH_2-C(=CH_2)-$;

A: ein heterocyclisches Kation der Formel 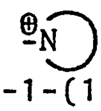, wie z.B. 3-Methyl-1-(1,2,3-triazolio), 2-Methyl-1-(1,2,3-triazolio), 4-Methyl-1-(1,2,4 triazolio), 3-Methyl-1-(1,3,4-triazolio), 3-Methyl-1-imidazolio, 1-Methyl-pyrrolio,2-Amino-1-pyrimidinio, 3-Methyl-1-pyrimidinio, 2-Hydroxyäthyl-1-pyrazolio, 2-Methyl-1-pyrazolio, 1-Pyridazinio, 3-Methyl-1-pyridazinio, Pyrazinio, 2,5-Dimethyl-1-pyrazinio, Thiazolio, 4-Methylthiazolio, Isothiazolio, Phthalazinio, 5,6,7,8-Tetrahydrophthalazinio, 3,4-Trimethylenpyridazinio, 4,5-Trimethylenpyridazinio, Cinnolinio, 5,6,7,8-Tetrahydrocinnolinio, Chinazolinio, 5,6,7,8-Tetrahydrochinazolinio, Chinoxalinio, 5,6,7,8-Tetrahydrochinoxalinio, N-Methylindolio;

Das erfindungsgemäße Verfahren zur Herstellung von Verbindungen der Formel I ist dadurch gekennzeichnet, daß man:

a) eine Verbindung der allgemeinen Formel II

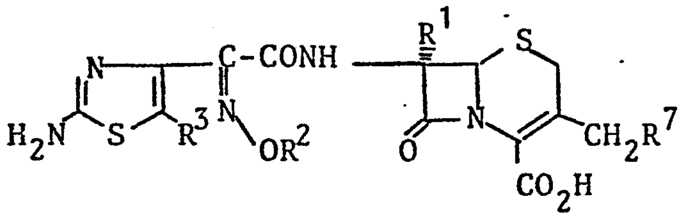

(II)

oder deren Salze, worin $R^1$, $R^2$ und $R^3$ die in Formel I genannte Bedeutung haben, und $R^7$ eine durch diejenige Base, die den Resten A der Formel I entspricht, austauschbare Gruppe bedeutet, mit dieser Base umsetzt und

α) eine gegebenenfalls vorhandene Schutzgruppe abspaltet und

ß) falls erforderlich, das erhaltene Produkt in ein physiologisch verträgliches Säureadditionssalz überführt,

oder

b) eine Verbindung der allgemeinen Formel III,

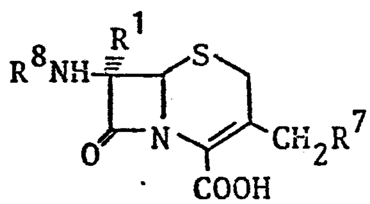

(III)

worin $R^1$ und $R^7$ die vorstehend für Formel II genannte Bedeutung haben und $R^8$ für Wasserstoff oder eine Aminoschutzgruppe steht, mit der Base, die dem in Formel I definierten Rest A zugrunde liegt, umsetzt unter Bildung der Verbindung der allgemeinen Formel IV,

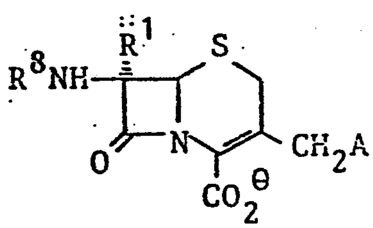

(IV)

in der $R^1$, $R^8$ und A die oben genannte Bedeutung haben und

α) eine gegebenenfalls vorhandene Aminoschutzgruppe abspaltet und

ß) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

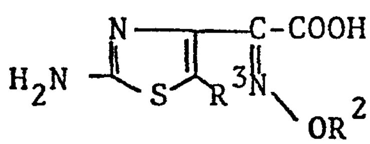

(V)

worin $R^3$ und $R^2$ die genannte Bedeutung besitzen, oder mit einem an der Carbonylgruppe aktivierten Derivat dieser Verbindung umsetzt und

a) eine gegebenenfalls vorhandene Schutzgruppe abgespaltet und

b) falls erforderlich, das erhaltene Produkt der allgemeinen Formel I physiologisch verträgliches Säureadditionssalz überführt.

Soll die Herstellung der Verbindungen der allgemeinen Formel I durch nucleophilen Austausch von $R^7$ in den Verbindungen der allgemeinen Formel II mit der Base, die dem in Formel I definierten Rest A zugrundeliegt, erfolgen, so kommen als Reste $R^7$ insbesondere in Betracht Acyloxyreste von niederen aliphatischen Carbonsäuren vorzugsweise mit 1 bis 4 C-Atomen, wie z.B. Acetoxy oder Propionyloxy, insbesondere Acetoxy, die gegebenenfalls substituiert sein können, wie z.B. Chlor-

acetoxy oder Acetylacetoxy. Für $R^7$ kommen auch andere Gruppen in Betracht, wie beispielsweise Halogen, insbesondere Chlor, Brom oder Jod, oder Carbamoyloxy.

Erfindungsgemäß werden bei der nucleophilen Austauschreaktion Ausgangsverbindungen der allgemeinen Formel II, in denen $R^7$ für Acetoxy steht, eingesetzt, oder deren Salze z.B. ein Natrium- oder Kaliumsalz. Die Reaktion wird in einem Lösungsmittel, vorzugsweise in Wasser oder in einem Gemisch aus Wasser und einem mit Wasser leicht mischbaren organischen Lösungsmittel, wie z.B. Aceton, Dioxan, Acetonitril, Dimethylformamid, Dimethylsulfoxid oder Äthanol durchgeführt. Die Reaktionstemperatur liegt im allgemeinen im Bereich von etwa 10 bis etwa 100 °C, vorzugsweise zwischen 20 und 80 °C. Die Basenkomponente wird in Mengen zugegeben, die zwischen etwa äquimolaren Mengen und einem bis zu etwa 15-fachen Überschuß liegt. Der Austausch des Restes $R^7$ wird durch Anwesenheit von Neutralsalzionen im Reaktionsmedium erleichtert. Insbesondere werden etwa 10 bis etwa 80 Äquivalente Kaliumjodid, Kaliumthiocyanat oder Natriumthiocyanat zugegeben. Die Reaktion wird vorteilhaft in der Nähe des Neutralpunktes, vorzugsweise bei einem pH-Wert im Bereich von etwa 5 bis 8 durchgeführt. Liegen in den Ausgangsverbindungen der allgemeinen Formel II geschützte Aminofunktionen vor, so eignen sich als Aminoschutzgruppen z.B. gegebenenfalls substituiertes Alkyl, wie beispielsweise tert.-Butyl, tert.-Amyl, Benzyl, p-Methoxy-benzyl, Trityl, Benzhydryl, vorzugsweise Trityl; Trialkylsilyl, wie beispielsweise Trimethylsilyl; gegebenenfalls substituiertes aliphatisches Acyl, wie z.B. Formyl, Chloracetyl, Bromacetyl, Trichloracetyl und Trifluoracetyl, vorzugsweise Formyl; oder gegebenenfalls substituiertes Alkoxycarbonyl, wie beispielsweise Trichloräthoxycarbonyl, Benzyloxycarbonyl oder tert.-Butyloxycarbonyl, vorzugsweise tert.-Butyloxycarbonyl und Benzyloxycarbonyl; oder Dimethylaminomethylen. Die Schutzgruppen sind literaturbekannt und können im Prinzip auch für den Schutz von OH und COOH-Funktionen verwendet werden. Die Schutzgruppe kann nach der Austauschreaktion in an sich bekannter Weise abgespalten werden, z.B. die Tritylgruppe mittels einer Carbonsäure, wie z.B. Essigsäure, Trifluoressigsäure, Ameisensäure oder die Benzyloxycarbonylgruppe hydrogenolytisch.

Die Reaktionsprodukte der Formel I können aus der Reaktionsmischung in üblicher Weise, z.B. durch Gefriertrocknen der Wasserphase, Chromatographie oder auch durch Ausfällen als schwerlösliches Salz, beispielsweise als Hydrojodid- oder Hydrothiocyanatsalz, isoliert werden.

Die nucleophile Austauschreaktion an Verbindungen der allgemeinen Formel II, kann auch so erfolgen, daß die Reaktion in Gegenwart der den Resten A entsprechenden Base und von Tri-$C_1$-$C_4$-alkyljodsilanen, wie z.B. Trimethyl- oder Triäthyljodsilan, vorgenommen wird. Dabei kann so verfahren werden, daß die Verbindung II zuerst mit Trimethyljodsilan nach den im folgenden genannten Reaktionsbedingungen zur Reaktion gebracht wird, die gebildete Verbindung II mit $R^7$ = J isoliert und anschließend mit der Base umgesetzt wird, oder die 3-$CH_2$J-Verbindung in situ durch Zugabe der Base zur Reaktion gebracht wird. Eine bevorzugte Ausführungsform ist in den deutschen Patentanmeldungen P 3 316 796.6, P 3 316 797.4 und P 3 316 798.2 beschrieben.
Sie besteht darin, daß zu einer Lösung oder Suspension der Verbindung II in einem geeigneten Lösungsmittel die dem Rest A entsprechende Base zugegeben wird, gefolgt von Trimethyljodsilan. Statt Trimethyljodsilan kann beispielsweise auch eine Reaktionsmischung aus Jod und Hexamethyldisilan, die vorher bei Temperaturen zwischen etwa 60 und 120 °C in literaturbekannter Weise zur Reaktion gebracht wurden,

 0137441

wobei Trimethyljodsilan entsteht, verwendet werden. Statt Trimethyljodsilan kann mit demselben guten Ergebnis auch Triethyljodsilan, das in literaturbekannter Weise hergestellt wird, verwendet werden.

Die Reaktion wird ausgeführt bei Temperaturen zwischen etwa -5 °C und +100 °C, vorzugsweise zwischen +10 °C und +80 °C.

Geeignete inerte aprotonische Lösungsmittel sind z.B. chlorierte Kohlenwasserstoffe, wie Methylenchlorid, Chloroform, Dichlorethan, Trichlorethan, Tetrachlorkohlenstoff, oder Niederalkylnitrile, wie Acetonitril oder Propionitril oder Frigene; insbesondere ist Methylenchlorid ein hervorragendes Lösungsmittel.

Die dem Rest A entsprechende Base wird in mindestens stöchiometrischer Menge bis zu einem zwanzigfachen Überschuß zugegeben, vorzugsweise wird mit solchen Mengen gearbeitet, daß die freiwerdende Jodwasserstoffmenge gebunden wird und noch mindestens 1 Mol, vorzugsweise 2-5 Mol der Base für die Substituion zur Verfügung stehen.

Da neben der auszutauschenden Gruppe $R^7$ in der Ausgangsverbindung II auch andere funktionelle Gruppe, wie z.B. die Carboxylgruppe, mit Trimethyljodsilan reagieren, wird letzteres in mindestens doppeltem bis zu fünfzehnfachen, vorzugsweise in drei- bis zehnfachem Überschuß zugegeben.

Derartige funktionelle Gruppen können auch durch Zugabe eines Silylierungsmittels wie z.B. Bistrimethylsilylacetamid, N-Methyl-N-trimethylsilyltrifluoracetamid, Bistrimethylsilyltrifluoracetamid, Trimethylchlorsilan, Hexamethyldisilazan, Bistrimethylsilylharnstoff, vorsilyliert werden, entweder ohne oder in Gegenwart einer

Base, vorzugsweise der gewünschten, der Gruppe A zugrundeliegenden Base in den vorstehend beschriebenen Mengen. Anschließend wird Trimethyljodsilan in mindestens stöchiometrischer Menge oder auch im Überschuß, vorzugsweise in einem doppelten bis zu einem zehnfachen Überschuß zugegeben.

Die Reaktionsprodukte der Formel I können beispielsweise nach Zugabe von Wasser oder wäßrigen Mineralsäuren, z.B. verdünnter HCl, HBr, HJ oder $H_2SO_4$, aus der wäßrigen Phase in üblicher Weise, z.B. durch Gefriertrocknen der Wasserphase, Chromatographie, oder Fällung durch Zugabe von organischen Lösungsmitteln, isoliert werden. Vorzugsweise werden die Reaktionsprodukte durch Ausfällen aus der wäßrigen Lösung in Form eines schwerlöslichen Salzes, beispielsweise eines Hydrojodidsalzes, isoliert.

Für den Fall, daß $R^7$ für eine Carbamoyloxygruppe steht, wird die Austauschreaktion analog durchgeführt. Steht $R^7$ für Brom, so erfolgt der Austausch in literaturbekannter Weise.

Nach der Verfahrensvariante b) werden die Verbindungen der allgemeinen Formel IV aus den Verbindungen der allgemeinen Formel III, z.B. der 7-Aminocephalosporansäure oder der 3-Jodmethyl-7-amino-ceph-3-em-4-carbonsäure, oder deren geschützte reaktionsfähige Derivate in analoger Weise wie vorstehend für die Verbindungen der Formel II beschrieben darstellt.

Die Verbindung der allgemeinen Formel IV wird anschließend mit Carbonsäuren der allgemeinen Formel V acyliert, wobei eine gegebenenfalls zur besseren Durchführung der Austauschreaktion vorhandene Aminoschutzgruppe $R^8$, beispielsweise eine tert.-Butyl, Benzyl, Trityl, Benzhydryl, Formyl, Trichloracetyl, Trifluoracetyl, Sulfo oder

Dimethylaminomethylen-Gruppe, in an sich bekannter Weise vorher abgespalten wird.

Werden die Carbonsäuren der allgemeinen Formel V selbst als Acylierungsmittel eingesetzt, so wird zweckmäßig in Gegenwart eines Kondensationsmittels, beispielsweise eines Carbodiimids, wie beispielsweise N,N'-Dicyclohexylcarbodiimid, gearbeitet.

Die Aktivierung der Carbonsäuren der allgemeinen Formel V kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie in der deutschen Patentschrift 28 04040 beschrieben wird.

Als aktivierte Derivate der Carbonsäuren der allgemeinen Formel V eignen sich insbesondere auch Halogenide, vorzugsweise Chloride, die in an sich bekannter Weise durch Behandlung mit Halogenierungsmitteln, wie z.B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel V eignen sich ferner die Anhydride und gemischten Anhydride, Azide und aktivierten Ester und Thioester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol, 6-Chlor-1-hydroxybenzotriazol, und 2-Mercaptobenzthiazol. Als gemischte Anhydride sind besonders geeignet solche mit niederen Alkansäuren, wie z.B. Essigsäure, und besonders bevorzugt solche mit substitutierten Essigsäuren, wie z.B. Trichloressigsäure, Pivalinsäure oder Cyanessigsäure. Besonders geeignet sind aber auch die gemischten Anhydride mit

Kohlensäurehalbestern, die man beispielsweise durch Umsetzung der Carbonsäuren der Formel V, in den die Aminogruppe geschützt ist, mit Chlorameisensäure-benzylester, -p-nitrobenzylester, -iso-butylester, -ethylester oder -allylester gewinnt. Die aktivierten Derivate können als isolierte Substanzen, aber auch in situ umgesetzt werden.

Im allgemeinen erfolgt die Umsetzung der Cephemderivate der allgemeinen Formel IV mit einer Carbonsäure der allgemeinen Formel V oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Ether, wie z.B. Diethylether, Tetrahydrofuran und Dioxan; Ketone, wie vorzugsweise Aceton und Butanon; Amide, wie vorzugsweise Dimethylformamid und Dimethylacetamid oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel IV mit einem in situ erzeugten aktivierten Derivat einer Carbonsäure der Formel V umgesetzt wird.

Es ist dabei nicht unbedingt erforderlich, die Verbindungen der allgemeinen Formel IV zu isolieren. Die Reaktion kann auch so durchgeführt werden, daß man Verbindungen der allgemeinen Formel III, beispielsweise 7-Aminocephalosporansäure oder 3-Jodmethyl-7-amino-ceph-3-em-4-carbonsäure oder deren geschützte reaktionsfähige Derivate in einem geeigneten Lösungsmittel mit der dem Rest A in der allgemeinen Formel IV entsprechenden Base umsetzt und die entstandene Verbindung der allgemeinen Formel I acyliert.

Die Umsetzung von Cephemverbindungen der Formel IV mit Carbonsäuren der Formel V bzw. deren aktivierten Derivaten kann in einem Temperaturbereich von etwa -80 °C bis etwa +80 °C, vorzugsweise zwischen etwa -30 °C und +50 °C, insbesondere jedoch zwischen etwa -20 °C und Raumtemperatur

erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z.B. Triethylamin oder Dimethylanilin; anorganische Basen, wie z.B. Kaliumcarbonat oder Natriumcarbonat; Alkylenoxide, wie z.B. Propylenoxid.

Auch die Anwesenheit eines Katalysators, wie z.B. von Dimethylaminopyridin, kann gegebenenfalls von Vorteil sein.

Liegt in den Verbindungen der allgemeinen Formel IV die Aminogruppe in Form eines reaktionsfähigen Derivates vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel IV mit einer Silylverbindung gebildet werden, wie z.B. Trimethylchlorsilan oder Bistrimethylsilylacetamid. Wird die Umsetzung mit einer solchen, an der Aminogruppe aktivierten Verbindung durchgeführt, so ist es zweckmäßig, die Reaktion in einem inerten Lösungsmittel, wie z.B. Methylenchlorid, Tetrahydrofuran oder Dimethylformamid, durchzuführen.

Als physiologisch verträgliche Säureadditionssalze der Verbindungen der allgemeinen Formel I seien beispielsweise erwähnt solche mit Chlorwasserstoffsäure, Bromwasserstoffsäure, Salpetersäure, Phosphorsäure, Schwefelsäure oder organischen Säuren, wie z.B. Methansulfonsäure, p-Toluolsulfonsäure oder Maleinsäure.

Die Verbindungen der allgemeinen Formel II und III sind literaturbekannt oder nach literaturbekannten Verfahren herstellbar.

Die erfindungsgemäß erhaltenen Verbindungen der allgemeinen Formel I und ihre physiologisch verträglichen Säureadditionssalze zeigen bemerkenswert gute antibakterielle Wirksamkeit sowohl gegen grampositive als auch gramnegative bakterielle Keime.

Auch gegen penicillinase- und cephalosporinasebildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I und ihre physiologisch verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel I mit einem oder mehreren pharmakologisch verträglichen Trägerstoffen oder Verdünnungsmittel, wie z.B. Füllstoffen, Emulgatoren, Gleitstoffen, Geschmackskorrigentien, Farbstoffen oder Puffersubstanzen vermischt und in eine

geeignete galenische Zubereitungsform bringt, wie beispielsweise Tabletten, Dragees, Kapseln, oder einer zur parenteralen Applikation geeigneten Suspension oder Lösung.

Als Träger- oder Verdünnungsmittel seien beispielsweise erwähnt Traganth, Milchzucker, Talkum, Agar-Agar, Polyglykole, Äthanol und Wasser. Puffersubstanzen sind beispielsweise organische Verbindungen wie z.B. N,N'-Dibenzyläthylendiamin, Diäthanolamin, Äthylendiamin, N-Methylglucamin, N-Benzylphenäthylamin, Diäthylamin, Tris(hydroxymethyl)aminomethan, oder anorganische Verbindungen, wie z.B. Phospatpuffer, Natriumcarbonat, Natriumbicarbonat. Für die parenterale Applikation kommen vorzugsweise Suspensionen oder Lösungen in Wasser mit oder ohne Puffersubstanzen in Betracht. Es istauch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen Säureadditionssalze liegen bei etwa 0,4 bis 20 g/Tag, vorzugsweise bei 0,5 bis 4 g/Tag, für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Die folgenden Ausführungsbeispiele für erfindungsgemäß herstellbare syn-Verbindungen dienen zur weiteren Erläuterung der Erfindung, schränken sie jedoch nicht darauf ein.

Beispiel 1 :

7-[2-(2-Aminothiazol-4-yl)-2-syn-methoxyimino-acetamido]-3-thiazoliomethyl-ceph-3-em-4-carboxylat:

Verfahren $a_1$:

Ein Gemisch aus 0.68 g (1.5 mmol) 7-[2-(2-Aminothiazol-4-yl)-2-syn-methoxyiminoacetamido]-cephalosporansäure, 10 g (60 mmol) Kaliumjodid, 1.3 g (1.53 mmol) Thiazol, 12 ml Wasser und 3 ml Aceton wird 4 Stunden auf 65° C erhitzt. Nach dem Abkühlen wird mit 80 ml Aceton verdünnt, von wenig Ungelöstem wird filtriert und die Lösung über 200 g Kieselgel (Merck 0.063-0.2 mm) chromatographiert. Elutionsfolge: 500 ml Aceton:Wasser (8:1); 500 ml Aceton:Wasser (5:1); die Titelverbindung wird sodann mit Aceton:Wasser (3:1) eluiert. Durch Gefriertrocknen der Produktfraktionen erhält man 0.15 g eines farblosen amorphen Feststoffs.

$^1$H-NMR (DMSO-$d_6$) : δ=3.2 - 3.7 (AB, 2H, $SCH_2$); 3.78 (s, 3H, $OCH_3$)
4.85 - 5.75 (m, 4H, $CH_2$-$\overset{\oplus}{N}$ und 2 Lactam-H)
6.67 (s, 1H, Thiazol); 7.22 (bs, 2H, $NH_2$);
8.25, 8.90 und 10.48 (je ein m, 3 Thiazol-H);
9.43 ppm (d, J=8Hz, NH)

Verfahren $a_2$:

Zu einem Gemisch aus 0.46 g (1 mmol) 7-[2-(2-Aminothiazol-4-yl)-2-syn-methoxyiminoacetamido]-cephalosporansäure, 0.72 g (8.5 mmol) Thiazol und 10 ml Methylenchlorid werden unter Kühlen 1.4 g (7 mmol) Trimethyljodsilan gegeben. Es wird 2 Stunden unter Rückfluß erhitzt, gekühlt und 2 ml 0.5 N Salzsäure zugegeben. Das Methylenchlorid wird im Vakuum entfernt, die dunkelgefärbte wässrige Lösung mit $NaHCO_3$ auf ph 6 gestellt und über eine "Lobar-C"-Kieselgelsäule (Fa, Merck, Darmstadt) mit Aceton:Wasser (2:1) chromatographiert. Nach dem Gefriertrocknen der Produktfraktionen erhält man 0.04 g amorphes Material, das in allen Eigenschaften mit dem vorstehend beschriebenen identisch ist.

Verfahren $a_3$:

2.05 g (4.5 mmol) 7-[2-(2-Aminothiazol-4-yl)-2-syn-methoxyiminoacetamido]-cephalosporansäure werden in 10 ml Chloroform suspendiert und nach Zugabe von 3 g (15 mmol) N-Methyl-N-trimethylsilyltrifluoracetamid 1 Stunde bis zur Lösung gerührt. Sodann werden 2.52 g (12.6 mmol) Trimethyljodsilan zugegeben und 15 Minuten bei Raumtemperatur gerührt. Die flüchtigen Bestandteile werden im Vakuum entfernt, der Rückstand in 10 ml Acetonitril gelöst und bei 0 °C 0.6 ml Wasser sowie 0.85 g (10 mmol) Thiazol zugegeben. Es wird 2 Stunden bei Raumtemperatur gerührt, 3 g Eis zugegeben und i.Vak. auf ein Volumen von 4 ml eingeengt. Der Rückstand wird über eine "Lobar C"-Kieselgelsäule mit Aceton:Wasser (2:1) chromatographiert. Nach dem Gefriertrocknen der Produktfraktionen erhält man 0,46 g amorphes Material, das in allen Eigenschaften mit dem vorstehend beschriebenen identisch ist.

Verfahren $b_1$:

0.4 g (2 mmol) 2-(2-Aminothiazol-4-yl)-2-syn-methoxyiminoessigsäure werden in 10 ml N,N-Dimethylformamid gelöst. Nach Zugabe von 0.28 g (2.1 mmol) 1-Hydroxybenztriazolhydrat und 0.41 g (2 mmol) N,N'-Dicyclohexylcarbodiimid wird 2 Stunden bei Raumtemperatur gerührt. Vom Dicyclohexylharnstoff wird filtriert und zum Filtrat eine Lösung von 0.6 g (2 mmol) 7-Amino-3-thiazoliomethyl-ceph-3-em-4-carboxylat in 10 ml Dimethylformamid und 1 ml Wasser gegeben. Nach 3 Stunden bei Raumtemperatur wird im Vakuum eingeengt und der Rückstand in 5 ml Wasser gelöst. Vom Ungelösten wird filtriert und die Lösung über eine Kieselgelsäule ("Lobar-C", Druck ca. 1 bar) mit Aceton:Wasser (2:1) chromatographiert. Die Produktfraktionen werden gefriergetrocknet. Ausbeute: 0.54 g amorpher Feststoff, der in allen Eigenschaften mit dem oben beschriebenen identisch ist.

Verfahren $b_2$:

0.68 g (2 mmol) 7-Amino-3-jodmethyl-ceph-3-em-4-carbonsäure werden in 30 ml N,N-Dimethylformamid suspendiert und 0.51 g (6 mmol) Thiazol zugegeben. Es wird 3 Stunden bei Raumtemperatur gerührt und anschließend eine Lösung von 2 mmol 2-(2-Aminothiazol-4-yl)-2-syn-methoxyiminoessigsäure-Hydroxybenztriazol-Aktivester (s. o. Verfahren $b_1$) in 10 ml N,N-Dimethylformamid zugegeben. Nach 2 Stunden bei Raumtemperatur wird i. Vak. eingedampft, der Rückstand in 5 ml Wasser gelöst, von wenig Ungelöstem wird filtriert und das Filtrat über eine "Lobar-C"-Säule mit Aceton:Wasser (2:1) chromatographiert. Nach dem Gefriertrocknen der Produktfraktionen erhält man 0.32 g amorphen Feststoff, der in allen Eigenschaften mit dem oben beschriebenen identisch ist.

Analog Beispiel 1 werden die nachfolgend aufgeführten Verbindungen erhalten, die der allgemeinen Formel I mit $R^1=R^3$=Wasserstoff und $R^2$=Methyl entsprechen und als Rest A den in der zweiten Spalte der Tabelle 1 angegebenen Substituenten tragen.

Tabelle 1

| Beispiel | A | Verfahren Ausb. % d.Th. | $^1$H-NMR: δ (ppm) in $CF_3CO_2D$ |
|---|---|---|---|
| 2 | 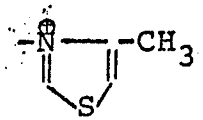 | $a_1$ (22)<br>$a_2$ (27)<br>$b_2$ (44) | 2.72 (s, 3H, $CH_3$); 3.53 und 3.83 (AB, J=19Hz, 2H, $SCH_2$); 4.26 (s, 3H, $OCH_3$); 5.0 - 6.2 (m, 4H, 2 Lactam-H und $CH_2\overset{\oplus}{N}$); 7.41 (s, 1H, Thiazol); 7.83 (m, 1H, Thiazol) 9.77 ppm (m, 1H, Thiazol) |
| 3 | 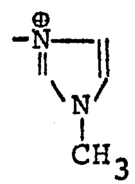 | $a_1$ (23) | 3.52 und 3.85 (AB, J=19Hz, 2H, $SCH_2$); 4.02 (s, 3H, $CH_3$); 4.25 (s, 3H, $OCH_3$); 4.75 - 6.25 (m, 4H, 2 Lactam-H und $CH_2\overset{\oplus}{N}$); 7.42 (s, 1H, Thiazol); 7.5 - 8.95 ppm (m, 3H, Imidazol) |
| 4 | 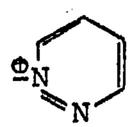 | $a_1$ (11)<br>$a_2$ (46) | 4.05 (m, 2H, $SCH_2$); 4.25 (s, 3H, $OCH_3$); 5.37 (d, J=5Hz, 1 Lactam-H); 5.68 und 6.28 (AB, J=14Hz, $CH_2\overset{\oplus}{N}$); 6.10 (d, J=5Hz, Lactam-H); 7.41 (s, 1H, Thiazol); 8.58 (m, 2H); 9.50 (m, 1H); 9.95 ppm (m, 1H); 4 Pyridazin-H |

| Beispiel | A | Verfahren Ausb. % d.TH. | $^1H$-NMR: δ (ppm) in $CF_3CO_2D$ |
|---|---|---|---|
| 5 | 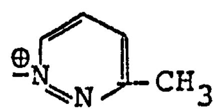 | $a_1$ (38) | 2.92 (s, 3H, $CH_3$); 3.8 - 4.2 (m, 2H, $SCH_2$); 4.26 (s, 3H, $OCH_3$); 5.25 - 6.42 (m, 4H, 2 Lactam-H und $CH_2\overset{\oplus}{N}$); 7.42 (s, 1H, Thiazol); 8.40 (m, 2H); 9.75 ppm (m, 1H): 3 Pyridazin-H |
| 6 | 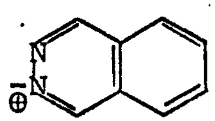 | $a_1$ (15) | 3.5 - 4.1 (m, 2H, $SCH_2$); 4.23 (s, 3H, $OCH_3$); 5.25 - 6.50 (m, 4H, 2 Lactam-H und $CH_2\overset{\oplus}{N}$); 7.41 (s, 1H, Thiazol); 8.4 - 8.8 (m, 4H); 9.72 (s, 1H); 10.80 (d, J=6Hz, 1H): 6 Phthalazin-H |
| 7 | 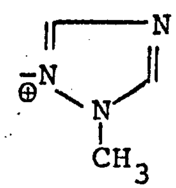 | $a_1$ (18)<br>$a_2$ (41) | 3.61 und 3.92 (AB, J=19Hz, 2H, $SCH_2$); 4.23 (bs, 6H, $CH_3$ und $OCH_3$); 4.95 - 6.20 (m, 4H, 2 Lactam-H und $CH_2\overset{\oplus}{N}$); 7.42 (s, 1H, Thiazol); 8.86 (s, 1H, Triazol); 9.75 ppm (bs, 1H, Triazol) |

Analog Beispiel 1 werden die nachfolgend aufgeführten Verbindungen erhalten, die der allgemeinen Formel I mit $R^1$=Wasserstoff entsprechen und als Reste $R^2$, $R^3$ und A die in Tabelle 2 angegebenen Substituenten tragen.

Tabelle 2

| Beispiel | $R^2$ | $R^3$ | A | Verfahren Ausb. % d.TH. | $^1H$-NMR: δ (ppm) in $CF_3CO_2D$ |
|---|---|---|---|---|---|
| 8 | $CH_3$ | Cl | 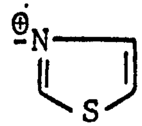 | $a_1$ (18)<br>$b_2$ (53) | 3.45 und 3.85 (AB, J=19Hz, 2H, $SCH_2$); 4.25 (s, 3H, $OCH_3$); 5.15-6.25 (m, 4H, 2 Lactam-H und $CH_2N^{\oplus}$); 8.15, 8.50 und 10.05 (je ein m, 3 Thiazol-H). |
| 9 | $C_2H_5$ | H | 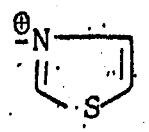 | $a_1$ (44) | 1.42(t, J=7Hz, 3H, $CH_2CH_3$); 3.53 und 3.84(AB, J=19Hz, 2H, $SCH_2$) 4.52 (q, J=7Hz, 2H, $CH_2CH_3$); 5.12-6.22 (m, 4H, 2 Lactam-H und $CH_2N^{\oplus}$); 7.42 (s, 1H, Thiazol); 8.13, 8.48 und 10.02 (je ein m, 3 Thiazol-H) |

| Beispiel | $R^2$ | $R^3$ | A | Verfahren Ausb. % d.Th. | $^1H$-NMR: δ (ppm) in $CF_3CO_2D$ |
|---|---|---|---|---|---|
| 10 | $CH_3$ | Cl | 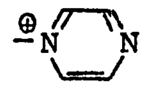 | $a_1$ (18) | 3.3-4.3 (m, 2H, $SCH_2$); 4.27 (s, 3H, $OCH_3$); 5.1-6.3 (m, 4H, $CH_2\overset{\oplus}{N}$ und 2 Lactam-H); 9.3-9.6 und 11.2-11.6 ppm ( je m, 4 Pyrazin-H) |
| 11 | $CH_2CONH_2$ | H | 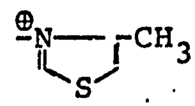 | $a_1$ (33) | 2.71 (s, 3H, $CH_3$); 3.50 und 3.82 (AB, J=19Hz, 2H, $SCH_2$); 4.95-6.25 (m, 6H, $OCH_2$, $CH_2\overset{\oplus}{N}$ und 2 Lactam-H); 7.45 (s, 1H, Thiazol), 7.82 (m, 1H, Thiazol), 9.78 ppm (m, 1H, Thiazol) |
| 12 | $-C(CH_3)_2COOH$ | H | 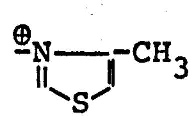 | $a_1$ (28) | 1.77 (s, 6H, zweimal $CH_3$); 2.75 (s, 3H, $CH_3$); 3.48 und 3.82 (AB, J=19Hz, 2H, $SCH_2$); 5.05-6.25 (m, 4H, $CH_2\overset{\oplus}{N}$ und 2 Lactam-H); 7.39 (s, 1H, Thiazol); 7.85 (m, 1H, Thiazol); 9.81 ppm (m, 1H, Thiazol) |

Analog Beispiel 1 werden die nachfolgend aufgeführten Verbindungen als amorphe Feststoffe erhalten, die der allgemeinen Formel I mit $R^1$=Wasserstoff entsprechen und als Reste $R^2$, $R^3$ und A die in Tabelle 3 angegebenen Substituenten tragen.

NMR-Spektren: Beispiele 13-19 in $CF_3CO_2D$
Beispiele 20-26 in DMSO-$D_6$

Tabelle 3

| Beispiel | $R^2$ | $R^3$ | A | Verfahren Ausb. % d. Th. | $^1$H-NMR: $\delta$ (ppm) |
|---|---|---|---|---|---|
| 13 | 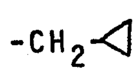 | H | 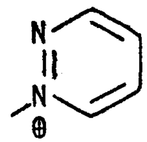 | $b_2$ (15) | 0.26-1.8 (m, 5H, Cyclopropyl-H); 4.05 (br, s, 2H, $SCH_2$); 4.3 (d, J =7Hz, 2H, $CH_2$-Cyclopropyl); 5.1-6.25 (m, 4H, $CH_2N^{\oplus}$ und 2 Lactam-H); 7.42 (s, 1H, Thiazol); 8.55 (2H), 9.5 (1H) und 9.95 ppm (1H): Pyrazin-H |
| 14 | 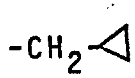 | H | 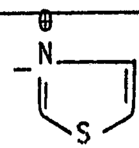 | $b_2$ (38) | 0.25-1.7 (m, 5H, Cyclopropyl-H); 3.1-4.4 (m, 4H, $SCH_2$ als AB und $CH_2$-Cyclopropyl als d bei 4.3); 5.15-6.25 (m, 4H, $CH_2N^{\oplus}$ |

| Beispiel | $R^2$ | $R^3$ | A | Verfahren Ausb. % d.Th. | $^1$H-NMR: δ (ppm) |
|---|---|---|---|---|---|
| | | | | | und 2 Lactam-H); 7.43 (s, 1H, Thiazol); 8.2, 8.52 und 10.1 ppm (je ein m, 3 Thiazol-H) |
| 15 | -$CH_2$-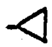 | H | 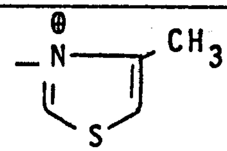 | $b_2$ (42) | 0.25-1.65 (m, 5H, Cyclopropyl-H); 2.73 (s, 3H, $CH_3$); 3.5-4.4 (m, 4H, $SCH_2$ als AB und $CH_2$-Cyclopropyl als d bei 4.3); 5.1-6.2 (m, 4H, $CH_2$-$\overset{\oplus}{N}$ und 2 Lactam-H); 7.44 (s, 1H, Thiazol); 7.83 und 9.87 ppm (je ein m, 2 Thiazol-H) |
| 16 | $CH_2CH=CH_2$ | H | 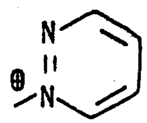 | $b_2$ (12) | 4.0 (br, s, 2H, $SCH_2$); 4.9 (m ähnl. d mit J=6Hz, 2H, Allyl-$CH_2$); 5.1-6.45 (m, 7H, $CH_2\overset{\oplus}{N}$, 2 Lactam-H und 3 Allyl-H); 7.45 (s, 1H, Thiazol); 8.58 (2H), 9.5 (1H) und 9.95 ppm (1H): Pyridazin-H |

0137441

| Beispiel | $R^2$ | $R^3$ | A | Verfahren Ausb. % d.Th | $^1H$-NMR: $\delta$ (ppm) |
|---|---|---|---|---|---|
| 17 | $CH_2CH=CH_2$ | H | 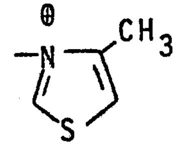 | $b_2$ (23) | 2.74 (s, 3H, $CH_3$); 3.25-4.15 (AB, J=19Hz, 2H, $SCH_2$); 4.92 (m ähnl. d mit J=6Hz, 2H Allyl-$CH_2$); 5.15-6.2 (m, 7H, $CH_2\overset{\oplus}{N}$, 2 Lactam-H und 3 Allyl-H); 7.42 (s, 1H, Thiazol); 7.84 und 9.8 ppm (je ein m, 2 Thiazol-H) |
| 18 | $CH_2C\equiv CH$ | H | 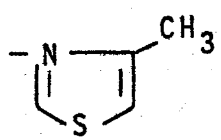 | $b_2$ (34) | 2.6 (t, J≈1.5 Hz, 1H, Propargyl-CH); 2.75 (s, 3H, $CH_3$); 3.4-4.1 (AB, J 18 Hz, 2H, $SCH_2$); 4.98 (d, J 1.5 Hz, 2H, Propargyl-$CH_2$); 5.2-6.2 (m, 4H, $CH_2\overset{\oplus}{N}$ und 2 Lactam-H); 7.42 (s, 1H, Thiazol); 7.85 und 9.78 ppm (je ein m, 2 Thiazol-H) |
| 19 | $CH_2C\equiv CH$ | H | 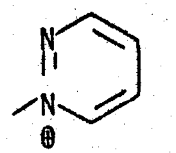 | $b_2$ (19) | 2.62 (t, J≈1.5 Hz, 1H, Propargyl-CH); 4.05 (br. s, 2H, $SCH_2$); 4.98 (d, J=1.5 Hz, 2H, Propargyl-$CH_2$); 5.2-6.3 (m, 4H, $CH_2\overset{\oplus}{N}$ und und 2 Lactam-H); 7.43 (s, 1H, Thiazol) 8.6 (2H), 9.55 (1H), 9.55 (1H) und 10.0 ppm (1H): Pyridazin-H) |

| Beispiel | $R^2$ | $R^3$ | A | Verfahren Ausb. % d.Th | $^1$H-NMR $\delta$ (ppm) |
|---|---|---|---|---|---|
| 20 | $CH_3$ | Cl | 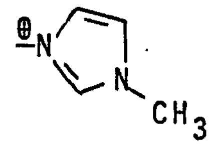 | $b_2$ (15) | 3.3 (s, 2H, $SCH_2$); 3.63 (s, 3H, $NOCH_3$) 3.8 (s, 3H, $NCH_3$); 5.0 (m, 3H, $CH_2\overset{\oplus}{N}$ und C-6-H); 5.6 (q, 1H, C-7-H); 7.33 (s, 2H, $NH_2$); 7.63 (1H), 8.0 (1H) und 9.25 (1H), Imidazol-H 9.4 (d, 1H, CONH) |
| 21 | $CH_3$ | Cl | 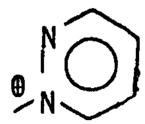 | $b_2$ (11) | 3.3 (s, 2H, $SCH_2$); 3.8 (s, 3H, $NOCH_3$); 5.0 und 5.7 (m, 4H, $CH_2\overset{\oplus}{N}$ + C-6-H+C-7-H) 7.3 (s, 2H, $NH_2$); 8.5-9.3 (m, 4H, Pyridazin-H) |
| 22 | $CH_3$ | Cl | 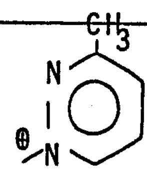 | $a_1$ (25) | 2.72 (s, 3H, $CH_3$); 3.3 (s, 2H, $SCH_2$); 3.8 (s, 3H, $NOCH_3$); 5.0 (d, 1H, C-6-H); 5.5 (AB, 2H, $CH_2\overset{\oplus}{N}$) 5.7 (q, 1H, C-7-H); 7.33 (s, 2H, $NH_2$) 8.3-9.5 (m, 3H, Pyridazin-H) |

| Beispiel | $R^2$ | $R^3$ | A | Verfahren Ausb. % d.Th | $^1H$-NMR $\delta$ (ppm) |
|---|---|---|---|---|---|
| 23 | $CH_3$ | Cl | 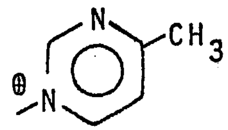 | $a_1$ (35) | 3.3 (s, 2H, $SCH_2$); 3.8 (s, 3H, $CH_3$); 3.9 (s, 3H, $NOCH_3$); 5.0 (d, 1H, C-6-H); 5.5 (m, 3H, C-7H+$CH_2\overset{\oplus}{N}$); 7.36 (s, 2H, $NH_2$) 9.5 (d, 1H, CONH); 8-10 (m, 3H, Pyrimidin-H) |
| 24 | $CH_3$ | Cl | 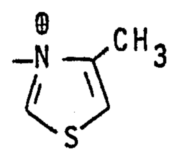 | $a_1$ (45) | 2.55 (d, 3H, $CH_3$); 3.3 (s, 2H, $SCH_2$); 3.8 (s, 3H, $OCH_3$); 5.0 (d, 1H, C-6-H); 5.23 (AB, 2H, $CH_2\overset{\oplus}{N}$); 5.6 (dd, 1H, C-7-H); 7.34 (s, 2H, $NH_2$); 7.98 (m, 1H, Thiazol C-5-H); 9.43 (d, 1H, Thiazol-C-2-H); 10.5 (d, 1H, CONH) |

0137441

| Beispiel | $R^2$ | $R^3$ | A | Verfahren Ausb. % d.Th. | $^1H$-NMR $\delta$ (ppm) |
|---|---|---|---|---|---|
| 25 | $CH_3$ | Cl | 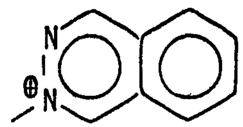 | $a_1$ (18) | 3.3 (s, 2H, $SCH_2$); 3.8 (s, 3H, $OCH_3$; 5.0 (d, 1H, C-6-H); 5.5 (m, 3H, C-7-H und $CH_2\overset{\oplus}{N}$); 7-7.5 (m, 6H, Phthalazin); 8.5 (s, 2H, $NH_2$); 9.4 (d, 1H, CONH) |
| 26 | $CH_3$ | Cl | 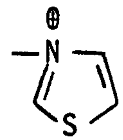 | $a_1$ (25) | 3.3 (s, 2H, $SCH_2$); 3.8 (s, 3H, $NOCH_3$); 5.0 (d, 1H, C-6-H); 5.27 (AB, 2H, $CH_2\overset{\oplus}{N}$); 5.63 (dd, 1H, C-7-H); 7.35 (s, 2H, $NH_2$) 8.27 (d, 1H, Thiazol-C-4-H); 8.97 (d, 1H, Thiazol-C-5-H); 9.43 (d, 1H, CONH); 10.53 (d, 1H, Thiazol-C-2-H) |

0137441

PATENTANSPRÜCHE:

1. Cephemderivate der allgemeinen Formel I

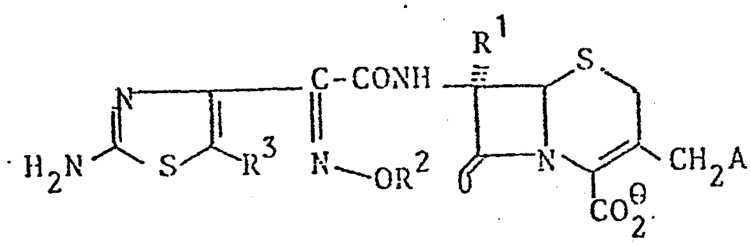

(I)

und deren physiologisch verträgliche Säureadditionssalze worin

$R^1$ Wasserstoff oder Methoxy

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $-(CH_2)_n-(\overset{R^4}{\underset{R^5}{C}})_m-R^6$, worin m und n jeweils für 0 oder 1 steht und

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfach substituiert sein können,

$R^6$ eine COOH-, CN- oder $CONH_2$-Gruppe,

$R^3$ Wasserstoff oder Halogen und

A ein ungesättigtes, gegebenenfalls substituiertes 5-6-gliedriges aromatisches heterocyclisches Kation der Formel $-N^{\oplus}$, das weitere N, O oder S-Atome im Ring

enthalten kann und an das zusätzliche Ringe ankondensiert sein können, wobei jedoch Pyridino und seine kondensierten Systeme ausgeschlossen sind, bedeutet, und in der die $R^2$ O-Gruppe in syn-Position steht.

2. Verfahren zur Herstellung von Cephemverbindungen der Formel I und ihrer physiologisch verträglichen Säureadditionssalze, dadurch gekennzeichnet, daß man

a) eine Verbindung der allgemeinen Formel II

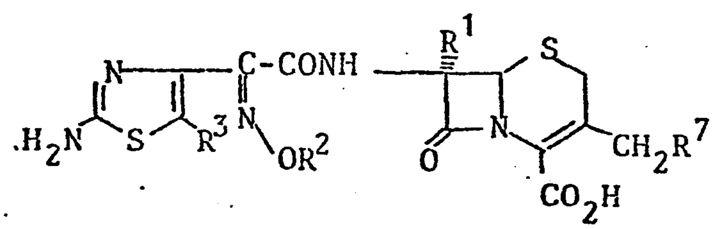

(II)

oder deren Salze, worin $R^1$, $R^2$ und $R^3$ die in Formel I genannte Bedeutung haben, und $R^7$ eine durch diejenige Base, die den Resten A der Formel I entspricht, austauschbare Gruppe bedeutet, mit dieser Base umsetzt und

α) eine gegebenenfalls vorhandene Schutzgruppe abspaltet und

ß) falls erwünscht das erhaltene Produkt in ein physiologisch verträgliches Säureadditionssalz überführt,

oder

b) eine Verbindung der allgemeinen Formel III,

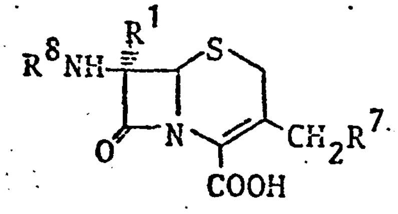

(III)

worin $R^1$ und $R^7$ die vorstehend für Formel II genannte Bedeutung haben und $R^8$ für Wasserstoff oder eine Aminoschutzgruppe steht, mit der Base, die dem in Formel I definierten Rest A zugrunde liegt, umsetzt unter Bildung der Verbindung der allgemeinen Formel IV,

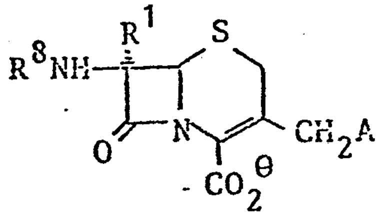

(IV)

in der $R^1$, $R^8$ und A die oben genannte Bedeutung haben und

α) eine gegebenenfalls vorhandene Aminoschutzgruppe abspaltet und

ß) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

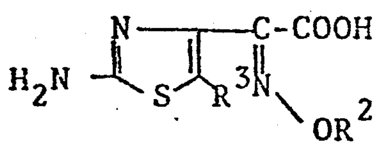

(V)

worin $R^3$ und $R^2$ die genannte Bedeutung besitzen, oder mit einem an der Carbonylgruppe aktivierten Derivat dieser Verbindung umsetzt und

a) eine gegebenenfalls vorhandene Schutzgruppe abgespaltet und

b) falls erwünscht, das erhaltene Produkt der allgemeinen Formel I in ein physiologisch verträgliches Säureadditionssalz überführt.

3. Verfahren gemäß Anspruch 2, dadurch gekennzeichnet, daß der nucleophile Austausch des Substituenten $R^7$ in Gegenwart von Neutralsalzionen, insbesondere von Jodid oder Thiocyanationen erfolgt.

4. Verfahren gemäß Anspruch 2, dadurch gekennzeichnet, daß der nucleophile Austausch des Substituenten $R^7$ in Gegenwart der dem Rest A zugrundeliegenden Base und von Tri-$C_1$-$C_4$-alkyljodsilan erfolgt.

5. Verfahren nach Anspruch 4, dadurch gekennzeichnet, daß das Tri-$C_1$-$C_4$-alkyljodsilan Trimethyl- oder Triethyljodsilan ist.

6. Gegen bakterielle Infektionen wirksame pharmazeutische Präparate, gekennzeichnet durch einen Gehalt an Cephemderivaten der allgemeinen Formel I.

7. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

8. Verwendung von Cephemderivaten der allgemeinen Formel I zur Bekämpfung bakterieller Infektionen.

0137441

Patentansprüche: Österreich

HOE 83/F 214

1. Verfahren zur Herstellung von Cephemverbindungen der Formel I

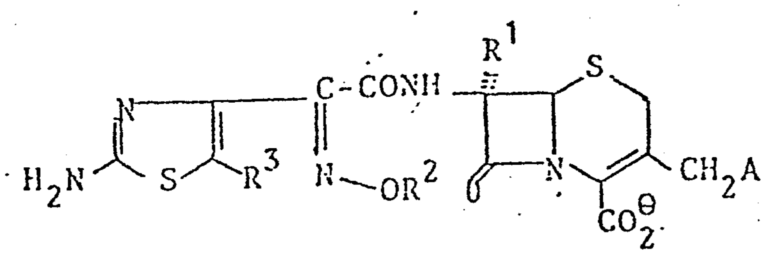

(I)

und deren physiologisch verträglichen Säureadditionssalzen worin

$R^1$ Wasserstoff oder Methoxy

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $-(CH_2)_n-(\overset{R^4}{\underset{R^5}{C}})_m-R^6$, worin m und n jeweils für 0 oder 1 steht und

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_5$-Cycloalkylidengruppe bilden, wobei die $C_1$-$C_4$-Alkyl- und die $C_3$-$C_7$-Cycloalkylidengruppe noch weiter ein- oder mehrfach substituiert sein können,

$R^6$ eine COOH-; CN- oder $CONH_2$-Gruppe,

$R^3$ Wasserstoff oder Halogen und

A ein ungesättigtes, gegebenenfalls substituiertes 5-6-gliedriges aromatisches heterocyclisches Kation der Formel 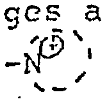, das weitere N, O oder S-Atome im Ring

enthalten kann und an das zusätzliche Ringe ankondensiert sein können, wobei jedcoh Pyridino und seine kondensierten Systeme ausgeschlossen sind, bedeutet, und in der die $R^2$ O-Gruppe in syn-Position steht, dadurch gekennzeichnet, daß man

a) eine Verbindung der allgemeinen Formel II

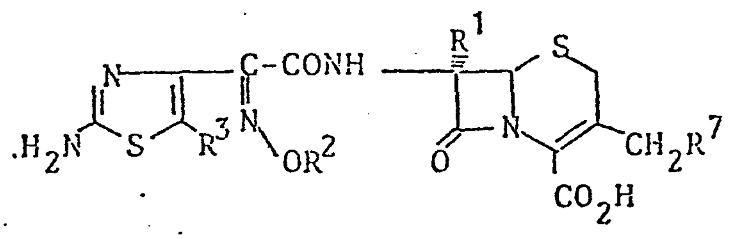

(II)

oder deren Salze, worin $R^1$, $R^2$ und $R^3$ die in Formel I genannte Bedeutung haben, und $R^7$ eine durch diejenige Base, die den Resten A der Formel I entspricht, austauschbare Gruppe bedeutet, mit dieser Base umsetzt und

α) eine gegebenenfalls vorhandene Schutzgruppe abspaltet und

ß) falls erwünscht das erhaltene Produkt in ein physiologisch verträgliches Säureadditionssalz überführt,

oder

b) eine Verbindung der allgemeinen Formel III,

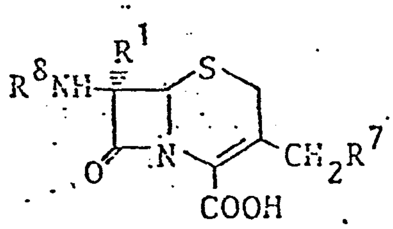

(III)

worin $R^1$ und $R^7$ die vorstehend für Formel II genannte Bedeutung haben und $R^8$ für Wasserstoff oder eine Aminoschutzgruppe steht, mit der Base, die dem in Formel I definierten Rest A zugrunde liegt, umsetzt unter Bildung der Verbindung der allgemeinen

Formel IV,

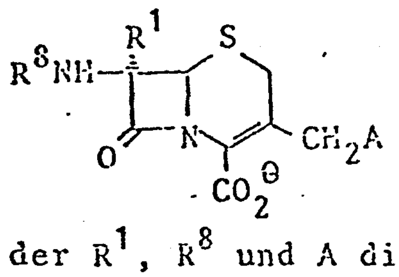

(IV)

in der $R^1$, $R^8$ und A die oben genannte Bedeutung haben und

α) eine gegebenenfalls vorhandene Aminoschutzgruppe abspaltet und

ß) die Verbindung IV, worin $R^8$ Wasserstoff bedeutet, entweder als solche oder in Form eines reaktionsfähigen Derivates mit einer 2-syn-Oxyiminoessigsäure der allgemeinen Formel V,

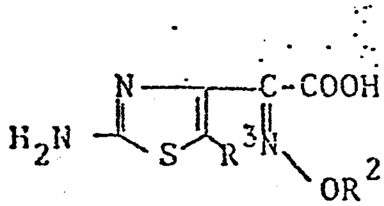

(V)

worin $R^3$ und $R^2$ die genannte Bedeutung besitzen, oder mit einem an der Carbonylgruppe aktivierten Derivat dieser Verbindung umsetzt und

a) eine gegebenenfalls vorhandene Schutzgruppe abgespaltet und

b) falls erwünscht, das erhaltene Produkt der allgemeinen Formel I in ein physiologisch verträgliches Säureadditionssalz überführt.

2. Verfahren gemäß Anspruch 1, dadurch gekennzeichnet, daß der nucleophile Austausch des Substituenten $R^7$ in Gegenwart von Neutralsalzionen, insbesondere von Jodid oder Thiocyanationen erfolgt.

3. Verfahren gemäß Anspruch 2, dadurch gekennzeichnet, daß der nucleophile Austausch des Substituenten $R^7$ in Gegenwart der dem Rest A zugrundeliegenden Base und von Tri-$C_1$-$C_4$-alkyljodsilan erfolgt.

4. Verfahren nach Anspruch 3, dadurch gekennzeichnet, daß das Tri-$C_1$-$C_4$-alkyljodsilan Trimethyl- oder Triethyljodsilan ist.